# EUROPEAN PATENT SPECIFICATION

(11) **EP 2 164 505 B1**
(45) Date of publication and mention of the grant of the patent: **17.09.2014**
(21) Application number: 08748014.1
(22) Date of filing: 28.05.2008
(51) Int. Cl.: A61K 35/48, A61K 35/12, A61P 11/00, A61K 35/50, A61K 31/01

(54) **TREATMENT OF CHRONIC LUNG DISEASE**
BEHANDLUNG VON CHRONISCHER LUNGENKRANKHEIT
TRAITEMENT D'UNE MALADIE CHRONIQUE DES POUMONS

(30) Priority: 28.05.2007 AU 2007902844 P
(43) Date of publication of application: 24.03.2010
(73) Proprietor: Monash University, Clayton VIC 3800 (AU)
(72) Inventor: JENKIN, Graham, Mount Waverley, VIC 3149 (AU); TROUNSON, Alan, Osborne, Ashburton, VIC 3147 (AU); WALLACE, Euan, Morrison, McKinnon, VIC 3204 (AU); MOODLEY, Yuben, South Caulfield, VIC 3162 (AU); MANUELPILLAI, Ursula, Aspendale Gardens, VIC 3195 (AU); ILANCHERAN, Sivakami, Clayton, VIC 3168 (AU)
(74) Representative: Purdylucey Intellectual Property
(86) International application number: PCT/AU2008/000753
(87) International publication number: WO 2008/144820

(56) References cited:
- WO-A1-03/078609
- WO-A2-2005/017117
- WO-A2-2007/141657
- WO-A2-2008/003042
- WO-A2-2008/036374
- US-A1- 2004 229 352
- PAROLINI O. ET AL.: 'Concise Review: Isolation and Characterization of Cells from Human Term Placenta: Outcome of the First International Workshop on Placenta Derived Stem Cells' STEM CELLS vol. 26, 2008, pages 300 - 311, XP002567088
- PAROLINI O. AND SONCINI M.: 'Human Placenta: a source of Progenitor/Stem Cells' JOURNAL OF REPRODUCTIVE MEDICAL ENDOCRINOLOGY vol. 3, no. 2, 2008, pages 117 - 126, XP008131909
- BAILO M. ET AL.: 'Engraftment Potential of Human Amnion and Chorion Cells Derived from Term Placenta' TRANSPLANTATION vol. 78, no. 10, 2004, pages 1439 - 1448, XP002568927
- MIKI T. ET AL.: 'Stem Cell Characteristics of Amniotic Epithelial Cells' STEM CELLS EXPRESS 04 August 2005, XP002410842
- KIM C.F. ET AL.: 'Identification of Bronchioalveolar Stem Cells in Normal Lung and Lung Cancer' JOURNAL OF CELL vol. 121, 17 June 2005, pages 823 - 835, XP008131911
- DELPLANQUE ET AL.: 'Epithelial stem cell-mediated development of the human respiratory mucosa in SCID mice' JOURNAL OF CELL SCIENCE vol. 113, 2000, pages 767 - 778, XP008125670

## Description

### FIELD OF THE INVENTION

The present invention relates to a method of cellular therapy for a lung disease or condition in a subject involving the administration of multipotent epithelial stem cells derived from amnion tissue. In a particular application, the method is used for the treatment of lung diseases and conditions such as chronic lung diseases including chronic obstructive pulmonary disease (COPD), acute lung conditions such as acute respiratory distress syndrome (ARDS), and ventilator associated lung injury (VALI).

### BACKGROUND OF THE INVENTION

Chronic lung diseases such as chronic obstructive pulmonary disease (COPD), cystic fibrosis (CF) and idiopathic pulmonary fibrosis (IPF) are a major global problem. COPD, in particular, is predicted to feature among the five most common causes of morbidity and mortality in the human population by 2020. As a group of diseases they are characterised by one or more of the following activities; infiltration of leukocytes into the lungs, interstitial lung inflammation, increased pro-inflammatory cytokine production, thickening of lung alveolar septae, hemorrhagic pneumonitis, distortion of normal lung architecture and fibrosis brought about by increased collagen deposition coupled with altered protease activity. Moreover, disease progression results in significant loss of lung tissue and scarring ultimately leading to the substantial morbidity and mortality associated with these chronic lung diseases.

Presently, treatment of chronic lung diseases is limited to symptom alleviation and/or prevention (eg bronchodilators such as β2-agonists and theophylline, and/or anti-inflammatory corticosteroids such as prednisone, are used to improve breathing capacity), and accordingly, the development of drugs or treatments to arrest, or preferably, reverse disease progression are greatly awaited. One possibility in this regard, would be to achieve restitution of the lung with appropriate stem cells that "traffic" to sites of lung injury and differentiate into endogenous lung tissue. To this end, the present applicant has studied the potential of multipotent human amnion epithelial stem cells (hAECs) (reviewed in Parolini, O., *et* al., 2008) for use in the treatment of chronic lung diseases and demonstrated, for the first time, that such cells, when cultured in Small Airway Growth Media (SAGM), adopt a lung cell phenotype as evidenced by the expression of lung specific markers and intra-cellular lamellar bodies. Further, the present applicant has found that when the hAECs were systemically administered into a mouse model of lung fibrosis (ie bleomycin-injured mice), they were located to the lung after 4 weeks and expressed the morphology and markers of alveolar epithelial cells. Moreover, it was observed that the multipotent hAECs also reduced inflammation by inhibiting the recruitment of macrophages through increased expression of macrophage inhibitory migratory factor (MIF) and down-regulation of macrophage inflammatory protein (MIP). This resulted in reduced expression of the inflammatory cytokines interleukin-1 (IL-1), interleukin-2 (IL-2), interleukin-6 (IL-6), tumour necrosis factor-alpha (TNF-α), inteferon-gamma (IFN-γ) and the pro-fibrotic transforming growth factor-beta (TGF-β). In addition, collagen concentrations in the lungs of the treated mice were significantly reduced by increased degradation by matrix metallo-proteinases (MMP-2 and MMP-9) and down-regulation of their endogenous inhibitors; that is, the tissue inhibitors of MMPs (TIMPs). Taken together, treatment with hAECs appears to address several pathways in the pathogenesis of lung injury and thereby offers considerable potential as the basis of an effective cellular therapy of lung diseases and conditions such as chronic lung diseases.

### SUMMARY OF THE INVENTION

In a first aspect, the present invention provides the use of multipotent amnion epithelial stem cells (AECs) in the preparation of a composition for cellular therapy for a lung disease or condition in a subject, wherein said composition comprises said AECs in combination with a pharmaceutically-acceptable carrier, as defined in the claims.

Preferably, the AECs used in the method of the disclosure are human amnion epithelial stem cells (hAECs). Further, the AECs used in the method of the disclosure are preferably AECs derived from amnion of term, or near term, placenta.

The lung disease or condition that may be treated with the method of the invention may be a chronic lung disease such as chronic obstructive pulmonary disease (COPD), cystic fibrosis (CF), idiopathic pulmonary fibrosis (IPF), or an acute lung condition such as acute respiratory distress syndrome (ARDS), or damage due to chemo- and/or radio-therapy, industrial accidents or exposure to toxic compounds or particulates (eg hot smoke). Alternatively, the lung disease or condition may be a lung condition arising from the use of a mechanical ventilator (ie VALI).

### BRIEF DESCRIPTION OF THE FIGURES

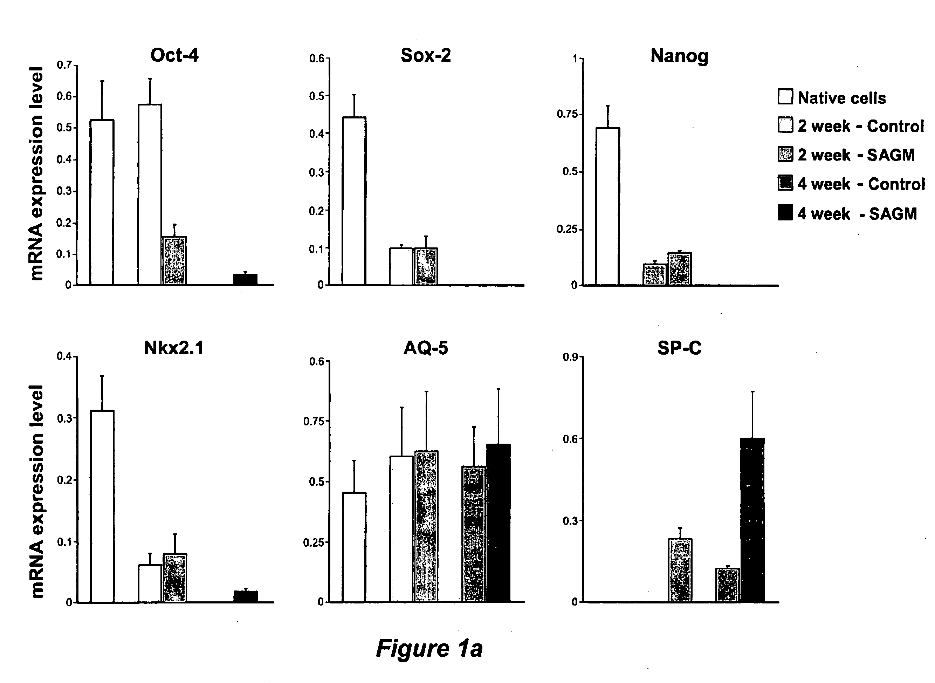

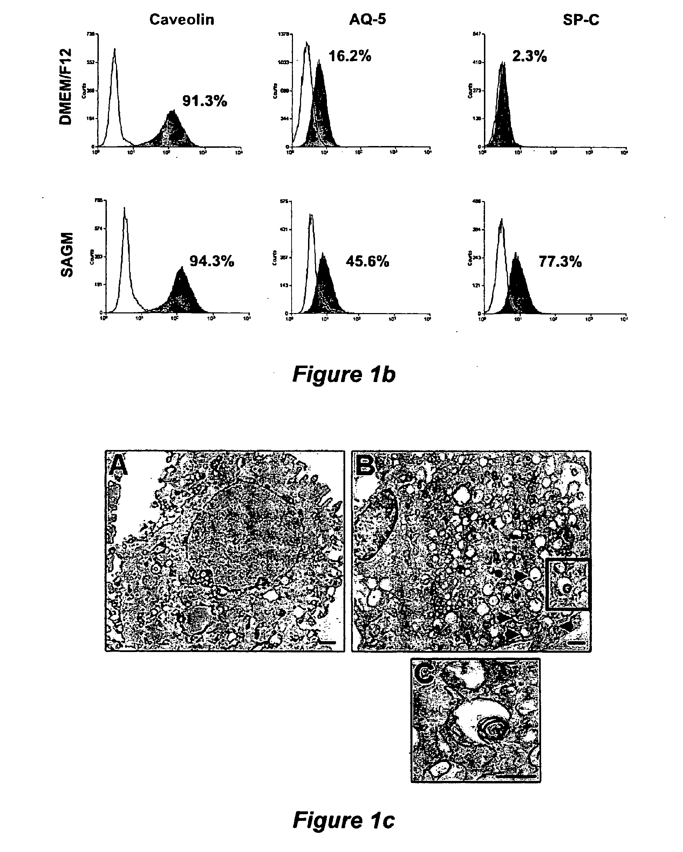

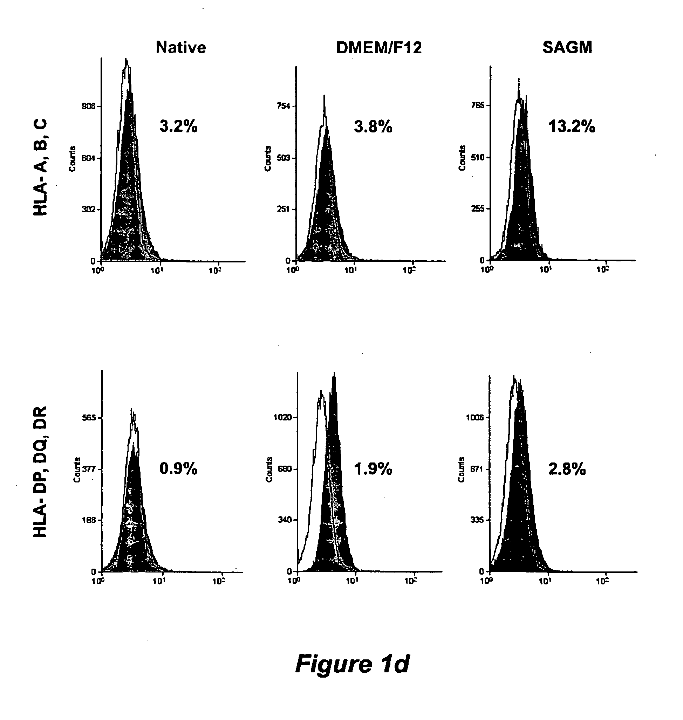

**Figure 1** a. shows the assessment, by quantitative polymerase chain reaction (PCR), of the expression of pluripotent markers by hAECs. The results shown demonstrate expression of Octamer-4 (Oct-4), Nanog, SRY-related HMG-box gene 2 (Sox-2) and thyroid transcription factor-1 (TTF-1, also known as Nkx 2.1), the latter being the earliest known marker of the respiratory lineage during development. Following culture in SAGM, the expression of these markers decreased while there was an increase in the expression of lung-specific surfactant protein C (SPC) as well as the epithelial markers aquaporin-5 (AQ-5). b. shows the increase in the percentage of SPC and Aquaporin-5 (AQ-5) producing cells following differentiation in SAGM. c. shows that hAECs cultured in SAGM also demonstrated morphological features of type II alveolar epithelial cells such as lamellar bodies (arrows). d. shows that hAECs express low levels of major histocompatability complex (MHC) class IA and II proteins both in freshly isolated cells and following *in vitro* differentiation in SAGM;

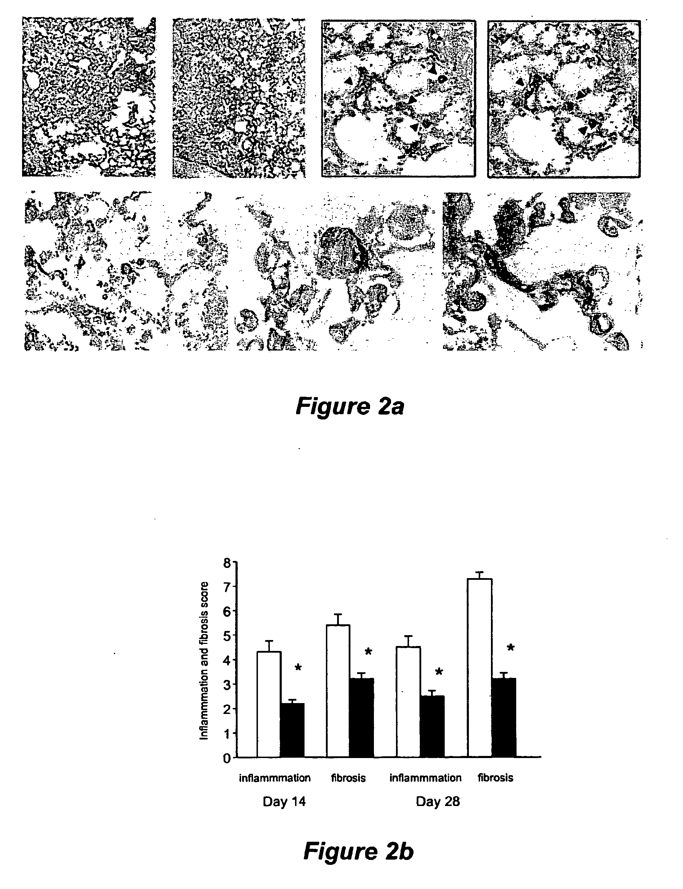

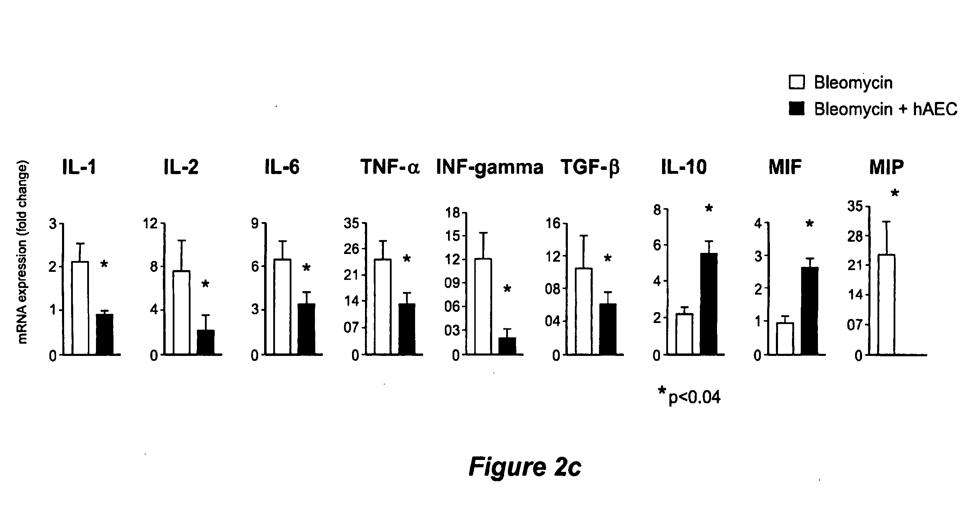

**Figure 2****.** a. shows the immunohistochemistry of lung sections harvested from mice following injection of hAECs into the tail vein 24h following intra-nasal bleomycin treatment. The sections demonstrate the presence of hAECs within the lung. The hAECs also acquired a morphology that closely resembled both type I (flattened) alveolar epithelial cells and type II (cuboidal) alveolar epithelial cells (lower panel). b. provides graphical results showing that the injection of hAECs into mice resulted in a reduction of the inflammation and fibrosis score of the lung following bleomycin-induced lung injury. c. provides graphical results showing that with the injection of hAECs into mice there was, following bleomycin-induced lung injury, a significant reduction in the expression of pro-inflammatory cytokines (eg IL-1, IL-6 and TNF-α) from lung transcripts (*p<0.05 comparing bleomycin+HAEC to bleomycin alone, by one way ANOVA); and

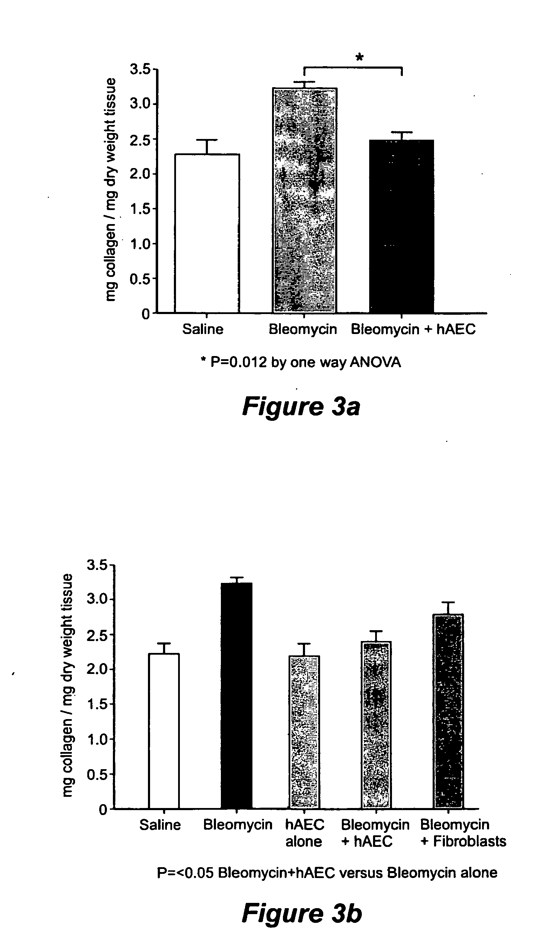

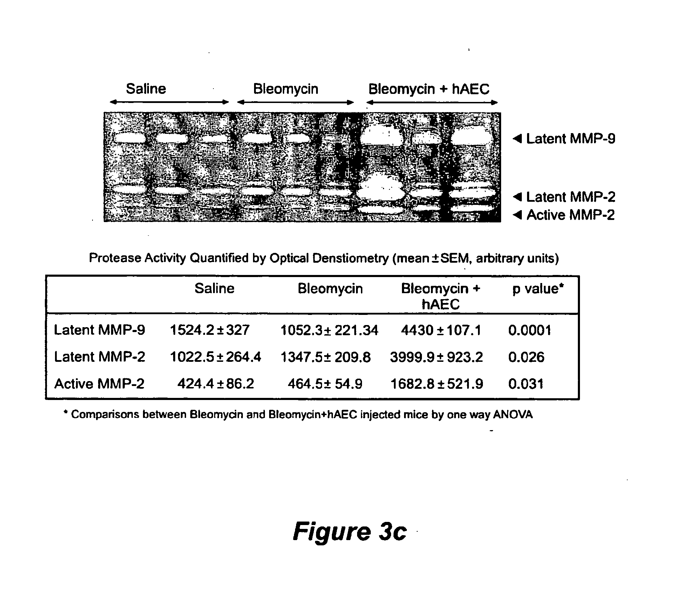

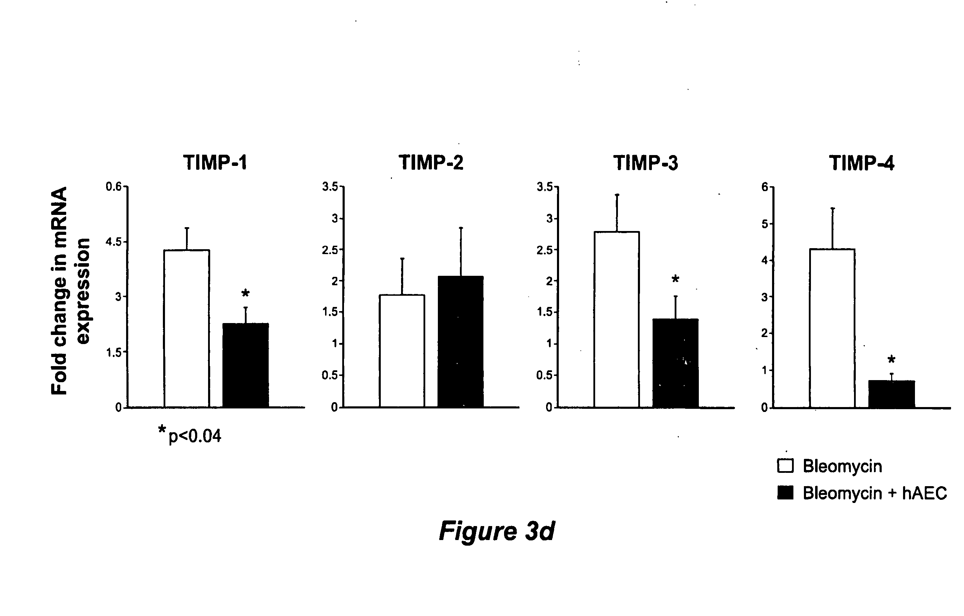

**Figure 3****.** a. shows graphical results showing that hAEC treatment (24 hr after bleomycin administration) resulted in a significant decrease in collagen deposition as compared to bleomycin treated mice alone. b. shows graphical results showing that hAEC treatment (2 weeks after bleomycin administration) also resulted in a significant decrease in the amount of deposited collagen as compared to bleomycin treated mice alone. c. shows the assessment of MMP-2 and -9 by zymography, the results demonstrating that there was a further significant increase in MMP activity in mice treated with bleomycin and hAECs. d. provides graphical results showing that transcripts of lung demonstrated reduced expression of tissue inhibitors of MMPs (TIMPs), the endogenous inhibitors of MMPs, following hAEC treatment (*p<0.05 compared to healthy controls, + p<0.05 comparing bleomycin+hAEC to bleomycin alone, by one way ANOVA).

### DETAILED DESCRIPTION OF THE INVENTION

The amnion and chorion form part of the embryonically-derived inner layers of the placenta with the maternally-derived decidua comprising the outer layer. While the chorion is a trophoblastic derivative, the amnion arises from the epiblast (ie at day 8 following fertilisation in humans) and, since the epiblast is also the origin of the three germ layers of the embryo, it has been suggested that cells of the amnion epithelium may be a ready source of pluripotent/multipotent stem cells (ie stem cells capable of differentiating into a range of cell types)(Ilancheran, S., *et al.,* 2007).

In work leading to the present invention, the applicant first found that at least a portion of the cells of the amnion epithelium express markers of pluripotency and proved to successfully differentiate *in vitro* into tissue representing all germ layers. Secondly, the applicant found that those cells could positively influence functional outcomes in chronic lung disease states; in particular, it was found that a reduction of lung inflammation and fibrosis could be achieved.

Thus, in a first aspect, the present disclosure provides a method of cellular therapy for a lung disease or condition in a subject, comprising administering to said subject a therapeutically effective amount of multipotent amnion epithelial stem cells (AECs).

For the purposes of the present invention, the AECs are at least capable of differentiating into one or more lung cell types (eg lung epithelial cells such as type II alveolar epithelial cells).

Preferably, the AECs used in the method of the disclosure are human amnion epithelial stem cells (hAECs).

Further, the AECs used in the method of the disclosure are preferably AECs derived from amnion of term, or near term, placenta. Near term human placenta can be regarded as placenta collected (eg by emergency or elective caesarean section, or near term deliveries) after about 34 weeks from conception.

The lung disease or condition that may be treated with the method of the disclosure may be selected from those characterised by lung inflammation and/or lung fibrosis.

In particular, the method of the disclosure can be used to treat a chronic lung disease such as chronic obstructive pulmonary disease (COPD), cystic fibrosis (CF) and idiopathic pulmonary fibrosis (IPF), or an acute condition such as acute respiratory distress syndrome (ARDS), or damage due to chemo- and/or radio-therapy, industrial accidents or exposure to toxic compounds or particulates (eg hot smoke).

Further, mechanical ventilation, which is commonly used in the management of critically ill subjects with respiratory failure, has been reported to be an important co-factor of ARDS and acute lung injury (ALI). In particular, it has been found that mechanical ventilation exacerbates lung inflammation in response to bacterial infection (Dhanireddy, S., *et al.,* 2006). Accordingly, the method of the invention can also be used to treat a lung condition arising from the use of a mechanical ventilator (ie ventilator associated lung injury (VALI)).

Moreover, the method of the disclosure can be used to treat pre-term infants lacking type II alveolar epithelial cells, as well as to treat and/or prevent foetal respiratory distress syndrome (RDS). Since the foetal lung is not fully developed until late in gestation, infants who are delivered prematurely are typically unable to breathe independently (thereby necessitating the use of a mechanical ventilator). The outcome for an infant of premature birth and/or respiratory support with a mechanical ventilator can consequently be the development of inflammation in the lung, diffuse injury to the alveoli and profound changes in lung mechanics; characteristic symptoms of RDS. Presently, foetal RDS is treated and/or prevented using "surfactant therapy" wherein synthetic or animal-derived surfactant is administered upon delivery (Morley, C.J., *et al.,* 1978). However, while such therapy has been a highly important advance in the care of preterm infants, it is considered that the method of the invention, especially when used in combination with a synthetic or animal-derived surfactant (as discussed further below), offers the potential for a greater therapeutic benefit than treatment by surfactant therapy alone. While not wishing to be bound by theory, the present applicant believes that this greater therapeutic benefit will be achieved through the known benefit of administering a synthetic or animal-derived surfactant in combination with the observed ability of AECs to bring about a reduction in lung inflammation, prevent fibrosis and differentiate into type II alveolar epithelial cells which secrete surfactant.

Also, the method of the disclosure can be used to treat a lung inflammation associated with smoking or industrial exposure to toxic compounds or particulates (eg hot smoke).

The method involves administering to the subject a therapeutically effective amount of multipotent AECs.

The mode of administration of the multipotent AECs will typically be by way of systemic transfusion, bronchoscopic or intra-nasal instillation, although it may also be satisfactory to implant the AECs (with or without a supporting substrate such as an implantable gel or solid scaffold material) directly into the lung at or near to a site of lung injury (eg sites of inflammation and/or fibrosis). The multipotent AECs will typically be administered in combination with a pharmaceutically-acceptable carrier (eg physiological saline).

Using systemic transfusion, it has been found that at least a portion of the tranfused multipotent AECs can migrate to the lung, particularly to a site(s) of lung injury, and present in the lung even after 4 weeks following administration. Once at the site(s) of lung injury, the multipotent AECs differentiate into lung epithelial cells to initiate repair and regeneration of lung tissue.

The term "therapeutically effective amount" is to be understood as referring to an amount of the multipotent AECs (ie a cell number) that will, at least, arrest the lung disease or condition being treated. Such an amount may vary considerably depending upon a range of factors such as the mode of administration, the age and/or body weight of the subject, and the severity of the lung disease or condition to be treated. However, typically, the amount will be in the range of about 1 x 10⁵ to 1 x 10¹⁰, more preferably, 1 x 10⁸ to 1 x 10¹⁰ multipotent AECs.

The multipotent AECs may be administered to the subject in combination with one or more active agents. For example, the AECs may be administered with one or more of the drugs presently used to alleviate and/or prevent symptoms of a lung disease or condition (eg a bronchodilator or anti-inflammatory corticosteroid). Alternatively, the AECs may be administered with an agent which causes a reduction in the expression or activity of one or more of the pro-inflammatory cytokines IL-1, IL-2, IL- 6, IFN-γ and TNF-α (eg a specific inhibitor or antagonist agent or specific antibody), and/or the expression or activity of the pro-fibrotic cytokine TGF-β (eg a specific TGF-β inhibitor or antagonist agent or specific anti-TGF-β antibody). The AECs may also be administered with synthetic or animal-derived lung surfactant (eg colfosceril palmitate-based products (such as Exosurf® marketed by GlaxoSmithKline plc, Brentford, Middlesex, United Kingdom) and surfactant extracted from bovine lung lavage fluid (such as Alveofact® marketed by Boehringer Ingelheim GmbH, Ingelheim, Germany)), to open unperfused areas of the lung to thereby aid access of the AECs to sites of injury.

The multipotent AECs are preferably characterised by the expression of one or more markers listed in Table 1 below. More preferably, the multipotent AECs are characterised by the expression of one or more pluripotent stem cell markers. For hAECs, the pluripotent markers will typically be selected from Oct-4, Nanog and Sox-2.

Further, the multipotent hAECs are preferably characterised in that they show low expression of MHC class IA and II proteins. This is preferred since it offers the potential use of the hAECs in subjects of divergent histocompatibility. The multipotent hAECs used in the method of the invention may, however, be autologous (and be sourced from preserved samples such as cryopreserved samples).

Multipotent AECs may be derived from amnion tissue by standard methods. For example, amnion tissue stripped from term placenta, can be treated with a suitable proteolytic enzyme (eg trypsin), and the purity of dispersed cells assessed by subjecting an aliquot to flow analysis (eg by FACS) for cytokeratin-7, an epithelial cytoskeletal protein found in AECs, and/or one or more of Oct-4, Nanog and Sox-2. Alternatively, the dispersed cells may be sorted/assessed by flow analysis for one or more relevant cell surface marker (eg SSEA-4). The dispersed/sorted cells may be stored for later use in accordance with standard methods. The dispersed/sorted cells may also be expanded by culturing under conditions which avoid cell differentiation (eg by culturing in a basal medium such as Dulbecco's Modified Eagle's Medium (DMEM)/F12 supplemented by animal-free serum or serum substitutes). When required, the dispersed/sorted, and optionally expanded, cells can be prepared for immediate administration to the subject by, for example, isolating the AECs from the culture/storage media and resuspending the AECs in a pharmaceutically-acceptable carrier.

Further, the multipotent AECs may be partially substituted with derivative cells; that is, cells which have been at least partially differentiated from the AECs (ie the invention may utilise "pre-differentiated" cells). Such derivative cells may be produced, for example, by culturing AECs under suitable conditions in a commercially available SAGM (eg SAGM products marketed by Lonza Group Ltd, Basel, Switzerland). Where the invention utilises derivative cells, the cells may be administered in an AEC:derivative cell ratio in the range of about 5:1 to about 1:5, more preferably about 4:1 to about 1:1.

Moreover, the multipotent AECs may be partially substituted with one or more other (ie non-derivative) cell types, including one or more other stem cell types. Where the invention utilises other cell types, the cells may be administered in an AEC:other cell ratio in the range of about 5:1 to about 1:5, more preferably about 4:1 to about 1:1.

However, while derivative cells or other cell types can be co-administered, the method of the invention, most preferably, utilises AECs only.

In a second aspect, the present disclosure provides the use of multipotent amnion epithelial stem cells (AECs) in the preparation of a composition for cellular therapy for a lung disease or condition in a subject, wherein said composition comprises said AECs in combination with a pharmaceutically-acceptable carrier.

Preferably, the multipotent AECs used in the use of the disclosure are human amnion epithelial stem cells (hAECs). Further, the AECs used in the method of the disclosure are preferably AECs derived from amnion of term, or near term, placenta.

In addition to the AECs, the composition may also comprise cells belonging to one or more other cell types including one or more other stem cell types and/or one or more wholly or partially differentiated cell types (eg derivative cells of AECs; that is, cells which have been at least partially differentiated from AECs).

The composition may also further comprise one or more active agents (eg a bronchodilator or anti-inflammatory corticosteroid).

The cellular therapy of the present invention offers the possibility of reducing, or at least arresting, lung diseases or conditions that have to date only been treated by the use of drugs to alleviate and/or prevent symptoms. Since the long term use of these drugs can also lead to serious side-effects (eg long term use of corticosteroids is associated with the development of several debilitating diseases and conditions including diabetes, osteoporosis and cataract formation), the identification and development of a cellular therapy for lung diseases or conditions, such as that of the present invention, is highly desirable.

The cellular therapy of the present invention is able to bring about a reduction in lung inflammation, prevent fibrosis and reduce collagen concentration in the lung following lung injury.

Accordingly, in a third aspect, the present disclosure provides a method of reducing inflammation in the lung of a subject, said method comprising administering to said subject a therapeutically effective amount of multipotent amnion epithelial stem cells (AECs).

And, in a fourth aspect, the present disclosure provides a method of preventing fibrosis in the lung of a subject, said method comprising administering to said subject a therapeutically effective amount of multipotent amnion epithelial stem cells (AECs).

Further, in a fifth aspect, the present disclosure provides a a method of reducing collagen concentration in the lung of a subject, said method comprising administering to said subject a therapeutically effective amount of multipotent amnion epithelial stem cells (AECs).

It will be appreciated by persons skilled in the art that numerous variations and/or modifications may be made to the disclosure defined in the first to fifth aspects above.

Further, the ability of AECs administered by systemic transfusion, bronchoscopic or intra-nasal instillation to migrate to the lung, provides a means or "vector" for delivering a therapeutic agent (eg a therapeutic protein or peptide, gene therapy agent or gene silencing agent) to the lung. For example, for the treatment of cystic fibrosis (CF), an exogenous polynucleotide molecule encoding cystic fibrosis transmembrane conductance regulator (CFTR) could be introduced into autologous AECs (ie lacking a functional *CFTR* gene) and thereafter administered to a subject suffering from CF, to provide a gene therapy for CF.

Accordingly, it is to be understood that the present disclosure extends to a method of cellular therapy for a lung disease or condition in a subject, comprising administering to said subject a therapeutically effective amount of multipotent amnion epithelial stem cells (AECs) comprising a therapeutic agent as described above, as well as to a composition therefor (ie a composition comprising AECs comprising a therapeutic agent as described above, in combination with a pharmaceutically-acceptable carrier) and, further, to the use of AECs comprising a therapeutic agent as described above in the preparation of a composition for cellular therapy for a lung disease or condition in a subject, wherein said composition comprises said AECs in combination with a pharmaceutically-acceptable carrier.

In order that the nature of the present invention may be more clearly understood, preferred forms thereof will now be described with reference to the following non-limiting examples.

### EXAMPLES

### Example 1 Human amnion epithelial cell abrogation of fibrosis and augmentation of lung repair

### Materials and Methods

### Isolation and culture of hAECs

Amnion was obtained from women undergoing elective caesarean section at term. hAECs were isolated and characterised using methods described previously (Ilancheran, S., *et al.,* 2007). Briefly, tissue was digested twice in 0.25% trypsin:EDTA solution (Invitrogen Corporation, Carlsbad, CA, United States of America) for 20 minutes. Trypsin was inactivated with foetal calf serum (FCS, Invitrogen) and dispersed cells washed in M199 medium (Invitrogen). Cells were then analysed by flow cytometry for cytokeratin-7 (Dako Denmark A/S, Glostrup, Denmark), an epithelial cytoskeletal protein found in hAECs. Batches with >99% cytokeratin-7 positive cells were plated onto type IV collagen (Roche Diagnostics, Basel, Switzerland) coated vessels and cultured in Small Airway Epithelial Basal Medium (SABM) (Clonetics, Walkersville, MD, United States of America) and supplements (ie retinoic acid, epidermal growth factor 1 (EGF-1), epinephrine, transferrin, insulin, tri-iodothyronine, bovine pituitary extract, bovine serum albumin (BSA), fatty acid-free serum and hydrocortisone) provided. Control cultures were grown in DMEM/F-12 medium (Invitrogen) with 10% human serum or FCS. Cultures were maintained for 4 weeks (n=12).

### Quantitative RT-PCR

Total RNA was isolated from hAECs (n=6) using RNeasy columns (Qiagen, Hilden, Germany). One µg of total RNA was converted to cDNA using the High-Capacity cDNA Archive Kit (Applied Biosystems, Foster City, CA, United States of America). The cDNA, diluted 1:20, was mixed with reagents (TaqMan Universal PCR Master Mix, Applied Biosystems), PCR primers and TaqMan probes for Oct-4, Sox-2, Nanog, Nkx2.1, SPC, AQ-5 and β-actin (Applied Biosystems #Hs01895061_u1, #Hs00602736_s1, #Hs02387400_g1, #NM_003317.3, #Hs00161628_m1, #Hs00387048_m1, respectively).

Oct-4 is a transcription factor that is highly expressed in the cells of the inner cell mass (ICM) and pluripotent embryonic carcinoma cells lines; low expression of Oct-4 yields the growth of trophoblastic cells only, while elevated expression of this transcription factor leads to differentiation of embryonic stem (ES) cells to embryonic and extra-embryonic tissue. Nanog is a homeodomain factor and is expressed *in vivo* in the morula and ICM of the epiblast. As such, it is a marker of pluripotent cells. Sox-2 is a transcription factor that is co-expressed with Oct-4 in the ICM as well as in ES cells and is essential for the function of Oct-4. Nkx-1 is also known as thyroid transcription factor-1 (TTF-1), and represents the earliest known marker of the respiratory lineage. SPC is highly specific for type II alveolar epithelial cells and Aquaporin-5 (AQ-5) is a lung (alveolar) epithelial cell markers.

The PCR parameters were: 95°C, 7sec and 60°C, 20sec for 40 cycles. Data was normalised against β-actin and expression levels, relative to the human embryonic stem cell line ES-1 (for Oct-4, Nanog, Sox-2, and Nkx2.1), or adult human lung (SPC and AQ-5) was calculated using the ΔΔCt method.

### Transmission electron microscopy (TEM)

Cells were fixed in 2.5% glutaraldehyde in 0.1M cacodylate buffer for 2 hours at room temperature (RT) and left overnight at 4°C. Cells were then post-fixed in 1% osmium tetroxide, dehydrated in graded acetone, infiltrated and embedded in Spurr's resin at 60°C for 24 hours. Ultra-thin sections (80nm) were thereafter stained with 3% uranyl acetate and Renoylds stain.

### Flow cytometry

hAECs (2.5×10⁵) were incubated with antibodies against caveolin (1:100), AQ-5 (1:50), SPC (1:50), human leukocyte antigen (HLA)-DP,-DQ,-DR (10µg/ml) or allophytocyanin (APC)-conjugated HLA-A,-B,-C (10µl), for 1 hour at RT. After several washes, cells were incubated with 1:10 diluted APC-conjugated donkey anti-rabbit (caveolin), donkey anti-goat (AQ-5, SP-C), or goat anti-mouse (HLA-DP,-DQ,-DR) secondary antibodies for 30 minutes at RT. Cells were washed to remove excess secondary antibodies and analysed by flow cytometry. Primary antibodies for caveolin, AQ-5 and SPC were purchased from Santa Cruz Biotechnology, Inc. (Santa Cruz, CA, United States of America) and HLA antibodies and secondary antisera from Becton, Dickinson and Company (Franklin Lakes, NJ, United States of America). Further flow cytometry analysis of the hAECs (including a popoulation (P5) that had been passaged 5 times) was undertaken, using similar methodology and monoclonal antibodies to the cell surface markers (Becton, Dickinson and Company) indicated in Table 2 below, in comparison with human bone marrow mesenchymal stem cells (hMSC). The percentage of the cells within the cell types that were "positive" for each marker was recorded.

### Injection of hAECs into SCID mice

Bleomycin sulphate in saline (0.15mg) was administered intranasally under weak ether anaesthesia to induce pulmonary fibrosis in 8-week-old Severe Combined Immune Deficient (SCID) mice (n=30). Twenty four hours after bleomycin instillation, mice were injected with hAECs (1 x 10⁶ in 0.2ml), via the tail vein, while control mice were injected with 0.2ml saline. Saline was administered intranasally to another group of mice (n=15); these mice received no further treatment. A parallel experiment where human lung fibroblast cells (1x10⁶) were used in place of hAECs was also carried out. Further, hAECs were also injected into healthy mice (n=12). Moreover, other SCID mice were injected, via the tail, with hAECs (1 x 10⁶ in 0.2ml saline) two weeks after bleomycin administration. In controls for these latter animals, lung fibroblasts (1 x 10⁶ in 0.2ml saline) isolated from lung resections were injected (via the tail) into SCID mice instead of hAECs 2 weeks after bleomycin administration. Animals were sacrificed two and four weeks post treatment (nb. four weeks only for the mice injected with hAECs/lung fibroblasts two weeks after bleomycin administration). Lung tissue was snap frozen and stored at -80°C and fixed in 10% formalin for paraffin embedding.

### Immunohistochemical analysis of lung tissue

Paraffin embedded tissue sections, 5µm in thickness, were dewaxed and rehydrated. Antigen retrieval was carried out by by heating in citrate buffer. After quenching endogenous peroxidase activity and blocking non-specific binding, sections were incubated with antibodies against human inner membrane mitochondria (IMM; 1:10, AbD Serotec, Kidlington, Oxfordshire, United Kingdom) and human SPC (1:50, Santa Cruz Biotechnology, Inc.) for 1 hour at 37°C. Isotype-matched mouse IgG2a and goat IgG were applied to sections serving as negative controls. Antibody binding was detected using horseradish peroxidase-labelled biotin-streptavidin secondary antibody (LSAB kit, Dako), and immunostaining visualised using 3,3'-diaminobenzidine chromogen. Co-localisation of IMM and SPC was assessed by examining serial tissue sections. For inflammation and fibrosis, sections were scored by the Ashcroft method (Ashcroft, T., *et al.,* 1988).

### Cytokine mRNA expression

Total RNA was extracted from lungs of SCID mice after two and four weeks of receiving saline, bleomycin and bleomycin+hAECs (n=5/group). Following DNAse treatment, 1µg of RNA was converted to cDNA with Superscript III (Invitrogen). The cDNA (diluted 1:20) was amplified with PCR primers against IL-1, IL-2, interleukin-4 (IL-4), IL-6, interleukin-10 (IL-10), interferon-gamma (INFγ), TNF-α, TGF-β, MIF and MIP. Cycling parameters used for quantitative PCR were: denaturation 95°C, 7sec, annealing 60°C, 7-15sec and extension 72°C, 15sec for 40 cycles. After normalising data to β-actin, expression relative to saline treated control mice was calculated by the ΔΔCt method.

### Collagen content

The collagen content in lungs of mice receiving saline, bleomycin and bleomycin+hAEC (n=6/group) was measured using the hydroxyproline assay as described previously (Hewitson, T.D., *et al.,* 2007). Briefly, lungs were lyophilised, hydrolysed in HCl and absorbance measured at 558nm. Collagen content was calculated by multiplying the hydroxyproline measurements by 6.94 and expressed as a proportion of dry weight of tissue (Gallop, P.M. and M.A. Paz, 1975).

### MMP activity and TIMP expression

MMPs were extracted from tissue obtained from the three groups of mice (n=6/group). MMP-2 and MMP-9 activity was assessed by gelatin zymography as described previously (Woessner, J.F. Jr, 1995). Zymographs were scanned on a densitometer (Gel Scan-710, Bio-Rad Laboratories, Inc., Hercules, CA, United States of America) and gelatinase activity quantified using Quantity-One software (Bio-Rad). The data was expressed as the mean±SEM.

TIMP-1, TIMP-2, TIMP-3 and TIMP-4 mRNA expression in lung tissue was measured by quantitative RT-PCR as described above. Reagents, primers and TaqMan probes were purchased from Applied Biosystems (#Mm00441818_m1, #Mm00441825_m1, #Mm00441826_m1, #Mm00446568_m1). After normalising data to β-actin, expression relative to saline instilled mice was calculated by the ΔΔCt method.

### Statistical analyses

Analysis of treatment effects between the different groups was performed using a one-way ANOVA analysis. P<0.05 was considered to be statistically significant.

### Results and Discussion

hAECs were derived from the placenta by stripping off the membrane and growing the hAECs in cell culture. These cells demonstrated clonality and, consistent with their epiblast derivation (Chambers I., *et al.,* 2003; and Mitsui K., *et al.,* 2003), expressed several markers of pluripotency, namely Oct-4, Nanog, Sox-1 and c-kit (Figure 1a and Table 1). The cells were not of vascular endothelial or haematopoetic lineage since they were "negative" for CD31, CD34 and CD45 markers (see Table 1). In comparative flow cytometry analysis, it was found that expression of cell surface markers by HAECs was considerably different to that of hMSC (most prominently in the CD90 marker that is characteristic of hMSCs), thereby indicating that the hAECs are of a distinct phenotype.

As part of a focus on lung differentiation and repair by hAECs, it was also demonstrated, by quantitative PCR, that the isolated hAECs expressed Nkx 2.1 (otherwise known as TTF-1), which is the earliest lineage marker of the developing lung (Figure 1a). Nkx. 2.1 is a critical transcription factor for branching morphogenesis and the formation of type II alveolar epithelial cells during lung development.

After noting the expression of Nkx 2.1 by the hAECs, the cells were then cultured in SAGM which had previously been shown to induce the differentiation of embryonic and umbilical cord blood-derived stem cells into type II alveolar epithelial cells of the lung (Berger, M.J., *et* al, 2006; and Ali, N.N., *et al.,* 2002). SAGM is primarily utilised in the maintenance of airway epithelial cells in culture and contains hydrocortisone, human epidermal growth factor (hEGF-1) and retinoic acid which are also factors present during lung development. HAECs cultured in SAGM for 2 and 4 weeks, demonstrated an increase in the gene expression of the lung specific gene surfactant protein C (SPC) (see Figure 1a). The increase in mRNA expression was translated into elevated protein expression of SPC shown by FACs analysis (Figure 1b). Ultra-structural analyses revealed that hAECs grown in SAGM for 4 weeks contained a distinct cell population demonstrating cytoplasmic organelles containing lamellar bodies, lipid filled vacuoles and surface microvilli formation that were indicative of type II alveolar epithelial cell differentiation. These features were not present in hAECs cultured in Dubelcos Modified Eagle Medium (DMEM)/F12. Type II alveolar epithelial cells constitute one third of the surface area of the lung but make up 66% of the cells in the distal lung and are responsible for the secretion of surface tension reducing surfactant protein. In addition, type II alveolar epithelial cells differentiate into the gas-exchanging type I alveolar epithelial cells during lung repair. Notably, hAECs demonstrate low expression of MHC antigens when freshly isolated and following *in vitro* differentiation, which widens the potential for the use of these cells for clinical application (see Figure 1c and 1d).

Having demonstrated the potential for hAECs to differentiate *in vitro* into a cell type that is central to lung repair, the ability of the hAECs to repair injured lung tissue *in vivo,* was then investigated.

The bleomycin-induced model of lung inflammation and fibrosis is well characterised and reflects the phases of lung injury in human subjects. The present applicant had previously established the model in SCID mice, and this allowed the evaluation of the functional role of human cells in augmenting lung repair. In particular, intra-nasally administered bleomycin induced an increase in both the inflammatory and fibrosis score on histology at both the 2 and 4 week time points (see Figure 2b). Further, there was an increase in the pro-inflammatory cytokines IL-1, IL-2, IL-6, IFNγ and TNF-α, as well as the pro-fibrotic cytokine TGF-β at the 2 week time-point when maximum injury and inflammation occurs.

In order to treat lung injury, hAECs were injected into the tail vein 24 hours following intra-nasal administration of bleomycin and were demonstrated in the lung by anti-human inner mitochondrial membrane (IMM) protein positive staining of cells at both 2 and 4 week time points. The anti-mitochondrial staining was negative in mouse lung tissue but present only in human controls (data not shown) thereby confirming the specificity and sensitivity of the antibody for the identification of human cells in the mouse lung. Also, when healthy mice were injected with hAECs, cells could not be detected in lung tissue 2 weeks post injection indicating that hAECs do not "traffic" to uninjured lung. Immunohistochemistry located the hAEC cells to the fibrotic and alveolar epithelial region of the lung (1 IMM-positive cell per 100 cells on high power field, n=8) as compared to no cells identified in uninjured healthy control mice subjected injected with hAECs. Moreover, 55 ± 5 % of hAECs in the alveoli of the lung also expressed SPC as evidenced by positive staining with a human specific antibody. Since the injected hAECs did not express SPC, the data indicates that the hAECs underwent differentiation into a respiratory lineage in the lung. Importantly, and despite the expression of some pluripotent markers, the hAECs did not form teratomas or tumours in the mice up to 4 weeks post-injection.

Notably, administration of hAEC by injection, reduced the inflammatory and fibrosis score at both 2 and 4 weeks (Figure 2c). Further, it was demonstrated that there was a significant reduction in macrophage number in the lung between the bleomycin treated mice and the mice treated with bleomycin and hAECs. In looking to elucidate the mechanism by which this reduction in macrophage number is achieved, the role of MIF and MIP was investigated. These proteins are known to be central to the absence of a rejection reaction when hAECs are used to repair damaged corneal epithelium (Hori, J. *et al.,* 2006; and Wang, M. *et al.,* 2006).

It was found that there was increased MIF and reduced MIP expression in lung homogenates that were treated with hAECs as compared to bleomycin controls, which appears to explain the reduced recruitment of macrophages to the sites of injury. Further, the reduction in macrophage recruitment may explain the down-regulation of the cytokines IL-1, IL-2, IL-6, IFN-γ and TNF-α, at 2 weeks by hAECs (Figure 2c); IL-1 is elevated during bleomycin lung injury and is usually secreted by monocytes and macrophages, promoting fibrosis by increasing fibroblast proliferation and collagen production. Indeed, the decrease in IL-1 by niacin and taurine during bleomycin lung injury parallels the improvement in symptoms of lung fibrosis (Gurujeyalakshmi, G., *et al.,* 2000). As such, the inhibition of IL-1 observed in this Example further points to the ability of hAECs to produce anti-fibrotic effects on the lung.

Notably, the cytokines IL-2, TNF-α and IFN-γ were significantly elevated in response to bleomycin in mouse strains prone to develop injury (C57/B16) as compared to the resistant strains of mice such as Balb/C, thus supporting the role for these cytokines in the progression of lung fibrosis. As such, inhibition of these cytokines by hAEC administration would serve to further reduce the development of fibrosis. In addition, several previous studies have demonstrated that the reduction in endogenous lung IFNγ and IFNγ knock-out mice are resistant to bleomycin injury. Moreover, TNFR-deficient mice are protective against bleomycin fibrosis by inhibiting the activation of TGF-β. As such, the significant reduction in TNF-α by HAEC administration provides even further support to the anti-fibrotic role of hAECs.

**Table 1 Characterisation of human amniotic epithelial stem cells**

| **Marker** | **Percentage (mean±SEM, n=4-7)** |
|---|---|
| Cytokeratin 7^{a} | 98.7±0.7 |
| Cytokeratin 8/18^{a} | 98.4±1.1 |
| Oct-4^{b} | 4.8±1.2 |
| Sox-2^{b} | 6.4±1.8 |
| SSEA-4^{b} | 98.6±0.6 |
| GCTM2^{b} | 97.4±1.3 |
| c-kit^{a} | 6.7±1.3 |
| HLA-A, -B, -C^{a} | 2.73±0.3 |
| HLA-DP, DQ- DR^{a} | nd |
| CD73^{a} | 95.3±1.2 |
| CD166^{a} | 91.9±4.2 |
| CD45^{a} | nd |
| CD31^{a} | nd |
| Nkx 2.1^{c} | present |

| | |
|---|---|
| ^{a}data from flow analyses; ^{b}data from immunocytochemistry; nd = not detected; ^{c}data from real time qPCR | |

**Table 2 Characterisation of human amniotic epithelial stem cells**

| **Marker** | **hMSC (%)** | **hAEC (%)** | **hAEC P5 (%)** |
|---|---|---|---|
| CD1b | 0 | 5 | 88 |
| CD1d | 0 | 4 | 21 |
| CD5 | 0 | 3 | 20 |
| CD6 | 0 | 4 | 26 |
| CD10 | 0 | 80 | 0 |
| CD13 | 100 | 5 | 17 |
| CD15 | 0 | 50 | 8 |
| CD15s | 0 | 15 | 44 |
| CD16 | 0 | 4 | 4 |
| CD24 | 0 | 1 | nd |
| CD26 | 0 | 72 | 10 |
| CD27 | 0 | 3 | 25 |
| CD29 | 100 | 94 | 98 |
| CD34 | 0 | 6 | 97 |
| CD36 | 0 | 8 | 20 |
| CD41b | 0 | 0 | 14 |
| CD42b | 0 | 4 | 17 |
| CD44 | 100 | 0 | nd |
| CD45 | 0 | 1 | 9 |
| CD45RA | 0 | 1 | nd |
| CD45RB | 0 | 3 | 0 |
| CD45RO | 0 | 4 | 23 |
| CD46 | 100 | 55 | 97 |
| CD47 | 100 | 100 | 99 |
| CD48 | 0 | 0 | 1 |
| CD49a | 100 | 0 | nd |
| CD49b(1) | 100 | 95 | 92 |
| CD49b(2) | 100 | 90 | 94 |
| CD49c | 100 | 99 | 98 |
| CD49e(1) | 100 | 9 | 0 |
| CD49e(2) | 100 | 2 | 5 |
| CD53 | 0 | 2.5 | nd |
| CD55 | 100 | 100 | 98 |
| CD56 | 0 | 3 | 21 |
| CD58 | 100 | 5 | 99 |
| CD59 | 100 | 100 | 69 |
| CD62P | 100 | 0 | nd |
| CD63 | 0 | 98 | 0 |
| CD66b | 0 | 2 | nd |
| CD66f | 0 | 0 | 71 |
| CD69 | 0 | 2 | nd |
| CD71 | 0 | 7 | nd |
| CD73 | 100 | 98 | 14 |
| CD77 | 0 | 30 | 88 |
| CD79b | 0 | 5 | 24 |
| CD81 | 100 | 35 | 99 |
| CD83 | 0 | 1 | 20 |
| CD84 | 0 | 1 | 0 |
| CD86 | 0 | 2 | nd |
| CD89 | 0 | 1 | nd |
| CD90 | 100 | 2 | nd |
| CD91 | 0 | 30 | 92 |
| CD95 | 100 | 15 | 100 |
| CD98 | 100 | 90 | 99 |
| CD100 | 0 | 1 | nd |
| CD104 | 0 | 87 | 95 |
| CD105 | 100 | nd | nd |
| CD106 | 0 | 2 | 11 |
| CD108 | 100 | 3 | 78 |
| CD109 | 0 | 15 | 0 |
| CD137(1) | 0 | 1 | nd |
| CD138 | 0 | 1 | 4 |
| CD140b | 100 | 0 | nd |
| CD141 | 0 | 8 | 60 |
| CD142 | 0 | 98 | 98 |
| CD 146 | 100 | 0 | nd |
| CD147 | 100 | 100 | 99 |
| CD150 | 0 | 5 | 0 |
| CD151 | 100 | 97 | 25 |
| CD153 | 0 | 1 | 3 |
| CD164 | 100 | 97 | 78 |
| CD165 | 100 | 0 | nd |
| CD166 | 100 | 28 | 77 |
| CD177 | 0 | 2.5 | nd |
| CD180 | 0 | 6 | 10 |
| CD181 | 0 | 3.5 | 24 |
| CD183 | 0 | 6 | 37 |
| CD184 | 0 | 1 | nd |
| CD195 | 0 | 6 | 56 |
| CD209 | 0 | 5 | 69 |
| CD210 | 0 | 1 | nd |
| CD212 | 0 | 2 | nd |
| CD227 | 0 | 98 | 75 |
| CD229 | 100 | 1.5 | nd |
| CD235 | 0 | 2 | nd |
| B7-H2 | 0 | 4 | 16 |
| CMRF-44 | 0 | 8 | 24 |
| B2-microglobulin | 100 | 83 | 50 |
| gd-TCR | 0 | 8 | 0 |
| CLA | 0 | 20 | nd |
| EGF-R | 100 | 30 | 76 |
| fMLP-R | | 2 | nd |
| fII-R | 0 | 99 | 86 |
| HLA-ABC | 100 | 93 | 41 |
| HLA-A2 | 100 | 0 | 0 |
| HLA-DQ | 100 | 15 | 62 |
| HLADRDPD | 0 | 1.5 | 9 |
| abTCR | 0 | 4 | 20 |
| M-calpain | 0 | 1.5 | 18 |
| N1-B1 | 0 | nd | nd |
| NP<-ALK/AL | 0 | 7 | nd |
| B glycoprotein | 0 | 0 | 22 |
| Invarient NK | 0 | 4 | nd |
| MUC2 | 0 | 2 | 2 |
| NGF-R | 0 | 1 | nd |
| PRR2 | 0 | 3.5 | nd |
| Common γ chain | 0 | 2 | nd |
| Stro-1 | nd | 0 | nd |

| | | | |
|---|---|---|---|
| nd = not determined; Also tested but recording 0% for hMSC and hAEC was: CD1a, CD2, CD3, CD4, CD7, CD8, CD9, CD11a, CD11b, CD11c, CD14, CD18, CD19, CD20. CD21, CD22, CD23, CD25, CD28, CD30, CD31, CD32, CD33, CD35, CD37, CD38, CD40, CD41a, CD42a, CD43, CD49d, CD49f, CD50, CD51/61, CD54, CD57, CD61, CD62e, CD62L, CD64, CD66, CD70, CD72, CD74, CD80, CD85J, CD87, CD88, CD94, CD97, CD99, CD99R, Cd103, CD110, CD117, CD123, CD134, CD135, CD137(2), CD140a, CD152, CD154, CD158a, CD158b, CD161, CD162, CD163, CD172b, CD182, CD200, CD201, CD206, CD220, CD221, CD226, CD244, CMRF-5, CLIP, Vb8-TCR, HLA-DR, IntegrinB7, LAIR-1, NK-G2d, NK-P46, ABC-G2, Blood Group A, Siglec-6, Siglec-7 and BS-TCR. | | | |

Turning to IL-6, this cytokine is secreted by fibroblasts, epithelial cells and macrophages, and has been found to be significantly elevated during bleomycin lung injury. It has been postulated that IL-6 acts in synergy with TNF-α to increase the levels of MIP, thereby enhancing the recruitment of macrophages and perpetuating inflammation and fibrosis.

TGF-β is a central cytokine in the pathogenesis of lung fibrosis. At day 14 following bleomycin lung injury, TGF-β is increased in macrophages, epithelial cells, fibroblasts and myofibroblasts. Indeed, several studies have demonstrated that TGF-β has a bimodal effect on fibroblast proliferation, stimulates monocyte production of cytokines and acts as a potent stimulator of collagen deposition (Kang, H.R., *et al.,* 2007). Moreover, several studies have conclusively shown that the amelioration of bleomycin fibrosis is dependent on the inhibition of TGF-β. Accordingly, the significant down regulation of TGF-β also indicates that hAECs have an anti-fibrotic role. In this example, it has been clearly demonstrated that hAECs reduce the cellular infiltration of the lung and reduce the pro-inflammatory cytokines IL-1, TNF-α and IL-6.

Further to the anti-inflammatory effects of hAECs, the role of hAECs in collagen deposition and fibrosis following bleomycin lung injury was investigated. Bleomycin induces excessive collagen deposition and fibrosis at 14 days post-injury resulting in the development of fibroblastic foci. These foci are characterised by the distortion of lung architecture, fibroblast and myofibroblast proliferation, collagen deposition and a reduction in functional lung. All of these are "generic" characteristics of fibrosis in all organs and show remarkable homology to pathology in human subjects.

Collagen deposition was measured by using a hydroxyproline assay. This showed an expected increase in collagen deposition in mice treated with bleomycin alone but a significant fall in the cohort subjected to bleomycin and hAEC injection (see Figures 3a and 3b). Notably, there was no influence of hAECs injected into healthy mice on collagen deposition. Further, it was found that the reduction of collagen was specific to hAECs since the injection of primary human lung fibroblasts into bleomycin exposed did not influence collagen levels. The results achieved with mice injected two weeks after treatment with bleomycin (see Figure 3b) indicates that hAECs abrogates fibrosis. This is significant since the maximum level of fibrosis in the bleoycin-induced mouse model occurs between 2-4 weeks; as such, the results indicate that hAECs injected at the time of maximal fibrosis are able to reduce or reverse collagen deposition without necessarily inhibiting the preceding inflammation that occurs maximally in the first two weeks following bleomycin lung injury.

Since matrix metalloproteinases (MMPs) are principally responsible for the degradation of extracellular matrix (ECM) proteins such as collagen, the expression of MMPs and their endogenous inhibitors (TIMPs) in homogenates of lung tissue was also assessed. Zymographic analysis demonstrated that MMP-2 and MMP-9 in bleomycin and hAEC lung homogenates were significantly elevated above bleomycin alone (Figure 3c). This appears to be a unique response to lung injury since there was no elevation of MMPs in lung samples from healthy controls injected with hAECs (data not shown). In addition, there was no additional increase in MMP levels following the injection of primary lung fibroblasts as compared to bleomycin alone (data not shown).

MMP-2 is responsible for the degradation of most ECM proteins and is also implicated in alveolar regeneration. Further, studies in human subjects have previously demonstrated that the up-regulation of MMP-2 was a strong predictor of a decrease in fibrosis in subjects with hypersensitivity pneumonitis (Selman, M. and A. Pardo, 1991). As such, it is considered that the significant up-regulation of MMP-2 is responsible for the reduction in collagen concentration within the lung.

In addition, there was a significant difference in MMP-9 expression between bleomycin alone versus bleomycin and HAEC treated mice. MMP-9 is instrumental in cleaving several ECM proteins but not directly implicated in active fibrosis since down regulation of MMP-9 did not influence collagen levels in bleomycin treated mice. However, the up-regulation of MMP-9 may be an additive degradative molecule during collagen deposition. Tissue inhibitors of MMPs (TIMPs) are the major endogenous inhibitors of MMPs and bind these molecules in a 1:1 stoichiometry. There are 4 homologues of TIMPs, TIMP-1-4, each having specific functions. Inhibition of TIMP-1 augmented the inflammatory response to bleomycin but not an increase in fibrosis. Similarly, TIMP-2 and TIMP-3 are also elevated in response to bleomycin. From lung transcripts, it was demonstrated that there was a down-regulation of TIMP-1, TIMP-3 and TIMP-4 in mice treated with hAECs at day 28 post bleomycin lung injury (Figure 3d). Moreover, there is a causal relationship between TIMP elevation and fibrosis since TIMP concentrations increased prior to the phase of collagen deposition in the bleomycin mouse model at days 14 to 28 post-injury. Also, mouse strains resistant to bleomycin fibrosis did not demonstrate an increase in TIMP-1 levels following injury. Thus, the reduction in TIMP levels accompanied by the increase in MMPs indicates the existence of a micro-environment that favours ECM degradation in response to HAEC injection, thereby reducing fibrosis.

Taken together, there are several mechanisms by which hAEC appears to directly augment lung repair. The differentiation of hAECs to type II alveolar epithelial cells, reduction in macrophage recruitment and inhibition of cytokine expression limits damage to the lung. In addition, the elevation in MMPs, with a consequent inhibition of TIMPs, abrogates fibrosis.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### REFERENCES

1. Ali, N.N., et al., Derivation of type II alveolar epithelial cells from murine embryonic stem cells. Tissue Engineering 8:541-550 (2002).
2. Ashcroft, T., et al., Simple method of estimating severity of pulmonary fibrosis on a numerical scale. J Clin Pathol 41:467-470 (1988).
3. Berger, M.J., et al., Differentiation of umbilical cord blood-derived multilineage progenitor cells into respiratory epithelial cells. Cytotherapy 8:480-487 (2006).
4. Chambers I., et al., Functional expression cloning of Nanog, a pluripotency sustaining factor in embryonic stem cells. Cell 113(5):551-552 (2003).
5. Dhanireddy S., et al., Mechanical ventilation induces inflammation, lung injury, and extrapulmonary organ dysfunction in experimental pneumonia. Laboratory Investigation 86:790-799 (2006).
6. Gallop, P.M. and M.A. Paz, Posttranslational protein modifications, with special attention to collagen and elastin. Physiological Reviews 55:418-487 (1975).
7. Gurujeyalakshmi, G., et al., Taurine and niacin block lung injury and fibrosis by down-regulating bleomycin-induced activation of transcription nuclear factor-kappaB in mice. J Pharmacol Exp Therap 293:82-90 (2000).
8. Hewitson, T.D., et al., Endogenous relaxin is a naturally occurring modulator of experimental renal tubulointerstitial fibrosis. Endocrinology 148:660-669 (2007).
9. Hori, J., et al., Immunological characteristics of amniotic epithelium. Cornea 25:S53-58 (2006).
10. Ilancheran S., et al., Stem cells derived from human fetal membranes display multi-lineage differentiation potential. Biol Reprod 77:577-588 (2007).
11. Kang, H.R., et al., Transforming growth factor (TGF)-beta 1 stimulates pulmonary fibrosis and inflammation via a Bax-dependent, bid activated pathway that involves matrix metalloproteinase-12. J Biol Chem 282:7723-7732 (2007).
12. Mitsui K., et al., The homeoprotein Nanog is required for maintenance of pluripotency in mouse epiblast and ES cells. Cell 113(5):631-642 (2003).
13. Morley, C.J., et al., The biochemistry and physiology of fetal pulmonary surfactant. In "Pre-term labour". Proc. V study group of Royal College of Obstetrics and Gynaecology. Eds. Anderson, A.B., Berad, R., Brundenell, J.M. and Dunn, P.M., pp 261-272.
14. Parolini, O., et al., Isolation and characterization of cells from human term placenta: outcome of the first international workshop on placenta derived stem cells. Stem Cells 26:300-311 (2008).
15. Selman, M. and A. Pardo, Potential role of proteases in pulmonary fibrosis. Annals of the New York Academy of Sciences 624:297-306 (1991).
16. Wang, M., et al., Immunogenicity and antigenicity of allogeneic amniotic epithelial transplants grafted to the cornea, conjunctiva, and anterior chamber. Investigative Ophthalmology & Visual Science 47:1522-1532 (2006).
17. Woessner, J.F. Jr, Quantification of matrix metaloproteinases in tissue samples. Methods in Enzymology 248:510-528 (1995).

## Claims

1. The use of multipotent amnion epithelial stem cells (AECs) in the preparation of a composition for cellular therapy for a lung disease or condition in a subject, wherein said composition comprises said AECs in combination with a pharmaceutically-acceptable carrier.

2. The use of claim 1, wherein the AECs are human amnion epithelial stem cells (hAECs).

3. The use of claim 2, wherein the hAECs are **characterised by** the expression of one or more markers selected from Oct-4, Nanog and Sox-2.

4. The use of any one of claims 1 to 3, wherein the AECs are derived from amnion of term, or near term, placenta, where near term placenta is a placenta collected, by emergency or elective caesarean section or near term deliveries, after 34 weeks from conception.

5. The use of any one of claims 1 to 4, wherein the AECs are autologous.

6. The use of any one of claims 1 to 5, wherein the lung disease or condition to be treated is chronic obstructive pulmonary disease (COPD), cystic fibrosis (CF) or idiopathic pulmonary fibrosis (IPF), or damage due to chemo- and/or radio-therapy, industrial accidents or exposure to toxic compounds or particulates.

7. The use of any one of claims 1 to 5, wherein the lung disease or condition to be treated is selected from the group consisting of acute respiratory distress syndrome (ARDS), acute lung injury (ALI), ventilator associated lung injury (VALI) and foetal respiratory distress syndrome (RDS).

8. The use of any one of claims 1 to 5, wherein the lung disease or condition is foetal respiratory distress syndrome (RDS) and the composition further comprises a synthetic or animal-derived surfactant.

9. The use of multipotent amnion epithelial stem cells (AECs) in the preparation of a composition for reducing inflammation in the lung of a subject.

10. The use of multipotent amnion epithelial stem cells (AECs) in the preparation of a composition for preventing fibrosis in the lung of a subject.

11. The use of multipotent amnion epithelial stem cells (AECs) in the preparation of a composition for reducing collagen in the lung of a subject suffering from a lung disease or condition selected from the group consisting of chronic obstructive pulmonary disease (COPD), cystic fibrosis (CF) or idiopathic pulmonary fibrosis (IPF), acute respiratory distress syndrome (ARDS), ventilator associated lung injury (VALI), foetal respiratory distress syndrome (foetal RDS) or damage due to chemo- and/or radio-therapy, industrial accidents or exposure to toxic compounds or particulates.

12. The use of any one of Claims 9 to 11, wherein the AECs are human amnion epithelial stem cells (hAECs).

13. The use of claim 12, wherein the hAECs are **characterised by** the expression of one or more markers selected from Oct-4, Nanog and Sox-2.

14. The use of any one of claims 9 to 13, wherein the AECs are derived from amnion of term, or near term, placenta, where near term placenta is a placenta collected, by emergency or elective caesarean section or near term deliveries, after 34 weeks from conception.

15. The use of any one of claims 9 to 13, wherein the AECs are autologous.

## Patentansprüche

1. Verwendung von multipotenten epithelialen Stammzellen aus dem Amnion (AECs = amnion epithelial stem cells) bei der Herstellung einer Zusammensetzung für die Zelltherapie für eine Lungenerkrankung oder ein Lungenleiden bei einem Subjekt, worin die genannte Zusammensetzung die genannten AECs zusammen mit einem pharmazeutisch verträglichen Träger umfasst.

2. Verwendung nach Anspruch 1, worin die AECs epitheliale Stammzellen aus humanem Amnion (hAECs = human amnion epithelial stem cells) sind.

3. Verwendung nach Anspruch 2, worin die hAECs durch die Expression von einem oder mehreren Markern, die aus Oct-4, Nanog und Sox-2 ausgewählt sind, gekennzeichnet sind.

4. Verwendung nach einem der Ansprüche 1 bis 3, worin die AECs aus Amnion der Plazenta zum Geburtstermin oder nahe des Geburtstermins stammen, worin die Plazenta nahe des Geburtstermins eine Plazenta ist, die im Notfall oder bei elektivem Kaiserschnitt oder Geburten nahe des Geburtstermins, nach 34 Wochen nach Empfängnis, gewonnen wird.

5. Verwendung nach einem der Ansprüche 1 bis 4, worin die AECs autolog sind.

6. Verwendung nach einem der Ansprüche 1 bis 5, worin die zu behandelnde Lungenerkrankung oder das zu behandelnde Lungenleiden Folgendes ist: chronisch obstruktive Lungenerkrankung (COPD), Mukoviszidose (CF) oder idiopathische Lungenfibrose (IPF) oder eine Schädigung durch Chemo- und/oder Strahlentherapie, industrielle Unfälle oder die Aussetzung gegenüber toxischen Verbindungen oder Partikeln.

7. Verwendung nach einem der Ansprüche 1 bis 5, worin die zu behandelnde Lungenerkrankung oder das zu behandelnde Lungenleiden aus der Gruppe ausgewählt ist, die aus akutem Atemnotsyndrom (ARDS), akuter Lungenschädigung (ALI), beatmungsassoziierter Lungenschädigung (VALI) und fetalem Atemnotsyndrom (RDS) besteht.

8. Verwendung nach einem der Ansprüche 1 bis 5, worin die Lungenerkrankung oder das Lungenleiden fetales Atemnotsyndrom (RDS) ist und die Zusammensetzung weiter ein synthetisches oder vom Tier stammendes Tensid umfasst.

9. Verwendung von multipotenten epithelialen Stammzellen aus dem Amnion (AECs) bei der Herstellung einer Zusammensetzung für die Verringerung von Entzündung in der Lunge eines Subjekts.

10. Verwendung von multipotenten epithelialen Stammzellen aus dem Amnion (AECs) bei der Herstellung einer Zusammensetzung für die Prävention von Fibrose in der Lunge eines Subjekts.

11. Verwendung von multipotenten epithelialen Stammzellen aus dem Amnion (AECs) bei der Herstellung einer Zusammensetzung für die Verringerung von Kollagen in der Lunge eines Subjekts, das an einer Lungenerkrankung oder einem Lungenleiden leidet, die aus der Gruppe ausgewählt sind, die aus Folgenden besteht: chronisch obstruktiver Lungenerkrankung (COPD), Mukoviszidose (CF) oder idiopathischer Lungenfibrose (IPF), akutem Atemnotsyndrom (ARDS), beatmungsassoziierter Lungenschädigung (VALI), fetalem Atemnotsyndrom (fetalem RDS) oder einer Schädigung durch Chemo- und/oder Strahlentherapie, industrielle Unfälle oder die Aussetzung gegenüber toxischen Verbindungen oder Partikeln.

12. Verwendung nach einem der Ansprüche 9 bis 11, worin die AECs epitheliale Stammzellen aus humanem Amnion (hAECs) sind.

13. Verwendung nach Anspruch 12, worin die hAECs durch die Expression von einem oder mehreren Markern, die aus Oct-4, Nanog und Sox-2 ausgewählt sind, gekennzeichnet sind.

14. Verwendung nach einem der Ansprüche 9 bis 13, worin die AECs aus Amnion der Plazenta zum Geburtstermin oder nahe des Geburtstermins stammen, worin die Plazenta nahe des Geburtstermins eine Plazenta ist, die im Notfall oder bei elektivem Kaiserschnitt oder Geburten nahe des Geburtstermins, nach 34 Wochen nach Empfängnis, gewonnen wird.

15. Verwendung nach einem der Ansprüche 9 bis 13, worin die AECs autolog sind.

## Revendications

1. Utilisation de cellules souches épithéliales amniotiques pluripotentes (CSÉA) dans la préparation d'une composition pour la thérapie cellulaire d'une maladie ou d'une affection pulmonaire chez un sujet, ladite composition comprenant lesdites CSÉA en combinaison à un véhicule pharmaceutiquement acceptable.

2. Utilisation de la revendication 1, dans laquelle les CSÉA sont des cellules souches épithéliales amniotiques humaines (CSÉAh).

3. Utilisation de la revendication 2, dans laquelle les CSÉAh sont **caractérisées par** l'expression d'un ou de plusieurs marqueurs sélectionnés parmi Oct-4, Nanog et Sox-2.

4. Utilisation de l'une quelconque des revendications 1 à 3, dans laquelle les CSÉA sont dérivées de la membrane amniotique d'un placenta à terme ou presque à terme, le placenta presque à terme correspondant à un placenta recueilli lors d'une opération césarienne d'urgence ou élective ou d'un accouchement presque à terme 34 semaines après la conception.

5. Utilisation de l'une quelconque des revendications 1 à 4, dans laquelle les CSÉA sont des cellules autologues.

6. Utilisation de l'une quelconque des revendications 1 à 5, dans laquelle la maladie ou l'affection pulmonaire à traiter est la bronchopneumopathie chronique obstructive (BPCO), la fibrose kystique (FK) ou la fibrose pulmonaire idiopathique (FPI) ou une lésion consécutive à une chimio- et/ou radiothérapie, à des accidents du travail ou à une exposition à des composés ou matières particulaires toxiques.

7. Utilisation de l'une quelconque de revendications 1 à 5, dans laquelle la maladie ou l'affection pulmonaire à traiter est sélectionnée dans le groupe consistant en les suivantes : syndrome de détresse respiratoire aiguë (SDRA), atteinte pulmonaire aiguë (ALI, pour *Acute Lung Injury*), lésion induite par la ventilation (VILI) et syndrome de détresse respiratoire (SDR) du nouveau-né.

8. Utilisation de l'une quelconque de revendications 1 à 5, dans laquelle la maladie ou l'affection pulmonaire est le syndrome de détresse respiratoire (SDR) du nouveau-né et dans laquelle la composition comporte également un surfactant synthétique ou d'origine animale.

9. Utilisation de cellules souches épithéliales amniotiques pluripotentes (CSÉA) dans la préparation d'une composition pour la réduction de l'inflammation dans les poumons d'un sujet.

10. Utilisation de cellules souches épithéliales amniotiques pluripotentes (CSÉA) dans la préparation d'une composition pour la prévention de la fibrose dans les poumons d'un sujet.

11. Utilisation de cellules souches épithéliales amniotiques pluripotentes (CSÉA) dans la préparation d'une composition pour la réduction du collagène dans les poumons d'un sujet souffrant d'une maladie ou affection pulmonaire sélectionnée dans le groupe consistant en les suivantes : bronchopneumopathie chronique obstructive (BPCO), fibrose kystique (FK), fibrose pulmonaire idiopathique (FPI), syndrome de détresse respiratoire aiguë (SDRA), lésion induite par la ventilation (VILI), syndrome de détresse respiratoire (SDR) du nouveau-né ou une lésion consécutive à une chimio- et/ou radiothérapie, à des accidents du travail ou à une exposition à des composés ou matières particulaires toxiques.

12. Utilisation de l'une quelconque des revendications 9 à 11, dans laquelle les CSÉA sont des cellules souches épithéliales amniotiques humaines (CSÉAh).

13. Utilisation de la revendication 12, dans laquelle les CSÉAh sont **caractérisées par** l'expression d'un ou de plusieurs marqueurs sélectionnés parmi Oct-4, Nanog et Sox-2.

14. Utilisation de l'une quelconque des revendications 9 à 13, dans laquelle les CSÉA sont dérivées de la membrane amniotique d'un placenta à terme ou presque à terme, le placenta presque à terme correspondant à un placenta recueilli lors d'une opération césarienne d'urgence ou élective ou d'un accouchement presque à terme 34 semaines après la conception.

15. Utilisation de l'une quelconque des revendications 9 à 13, dans laquelle les CSÉA sont des cellules autologues.
